# EUROPEAN PATENT SPECIFICATION

(11) **EP 2 538 938 B1**
(45) Date of publication and mention of the grant of the patent: **24.04.2019**
(21) Application number: 11706319.8
(22) Date of filing: 25.02.2011
(51) Int. Cl.: A61K 31/19, A61K 31/194, A61K 45/06, A45D 29/04, A45D 29/18, A45D 34/06, A61M 35/00, A61P 17/00, A61P 31/10, A61K 47/10, A61K 47/18, A61K 47/20, A61K 47/36, A61K 9/00, A61K 9/70

(54) **FUNGAL NAIL TREATMENT COMPOSITION**
ZUSAMMENSETZUNG ZUR BEHANDLUNG VON NAGELPILZ
COMPOSITION DE TRAITEMENT D'INFECTION FONGIQUE DES ONGLES

(30) Priority: 26.02.2010 GB 201003336
(43) Date of publication of application: 02.01.2013
(73) Proprietor: LRC Products Limited, Slough, Berkshire SL1 3UH (GB)
(72) Inventor: JOHNSON, Neil, Manchester Greater Manchester M41 7HA (GB)
(74) Representative: O'Brien, Niall James
(86) International application number: PCT/GB2011/050379
(87) International publication number: WO 2011/104562

(56) References cited:
- WO-A1-99/53913
- WO-A1-2011/019317
- WO-A2-2004/010952
- WO-A2-2009/156682
- AT-B- 414 096
- GB-A- 2 358 627
- US-A1- 2003 012 749
- US-A1- 2003 235 607
- US-A1- 2005 181 999
- US-A1- 2008 188 568
- US-B1- 6 281 239
- DATABASE WPI Week 200830 Thomson Scientific, London, GB; AN 2008-E29029 XP002635601, -& CN 101 099 857 A (GUIZHOU SHENQI GROUP HOLDING CORP LTD) 9 January 2008 (2008-01-09)

## Description

The present invention relates generally to fungal conditions and their treatment, and in particular to a composition for use in treating such conditions, particularly fingernail and toenail conditions.

Fungal nail (or onychomycosis) is estimated to affect approximately 2% to 13% of the population of Europe and North America, with the prevalence increasing with age. The condition arises due to the invasion of the nail bed by dermatophytes (a group of three types of fungus that commonly cause skin disease in animals and humans) species, typically Trichophyton rubrum or Trichophyton mentagrophytes, and manifests itself as yellow, brown or white discoloured patches in the nail that emanate from the underside of the nail. Depending on the site of dermatophyte invasion, several different patterns of onychomycosis are observed. The most common pattern arises from invasion of the distal or lateral nail margins (distolateral subungal onychomycosis). Less commonly, the nail plate surface is invaded directly, resulting in white islands (white superficial onychomycosis). Secondary symptoms include nail plate thickening and ridging. Onychomycosis is a degenerative condition and, if untreated, the invading dermatophytes spread proximally through the nail plate and bed, leading to breakdown and eventual detachment of the nail plate.

Whilst onychomycosis is classified as a medical condition by many, the primary concern of sufferers is the negative cosmetic impact of the ailment. Many sufferers feel embarrassment at the visual appearance of their fingernails or toenails and wish to regain the normal nail appearance. Most fungal nail treatment products act upon the root cause of the condition, that is, the dermatophytes, in an attempt to achieve a mycological cure. However, the outcomes of these treatments are in fact the cosmetic benefits of the nail plate returning to a normal appearance, as opposed to actual permanent, effective treatment of the infection.

There are currently several options available to sufferers of fungal nail to help improve the appearance of their toenails and fingernails. These can be broadly classified into three groups: Oral systemic medication, topical medicines, and medical devices. All of these options have potential disadvantages for the use. With regards to oral systemic medication, fungal nail is treated using systemic antifungals such as terbinafine, itraconazole or griseofulvin, which are taken orally and are absorbed into the circulatory system, eventually reaching the nail bed and nail plate. The treatment route can have low efficacy, and therefore the treatment has to be continued for a substantial period of time; in some cases it can also lead to serious side effects such as liver damage.

Topical drugs or medicines are sold over-the-counter and are typically provided in a nail varnish or lacquer which forms an impervious film over the nail plate. Over time the drug diffuses from the varnish or lacquer, into the nail plate and then towards the nail bed where it kills dermatophytes by direct pharmacological action on them. For example, WO 2007/147052 and US 2005/181999 disclose topical compositions for the treatment of fungal nail infection, which compositions comprise biologically active agents. The biologically active agents may be antifungal agents, antibacterial agents, anti-inflammatory agents or antiviral agents.

WO 2004/010952 describes methods and compositions for the treatment of pathological conditions of the dermis and dermal structures of animals and humans. In particular, WO 2004/010952 describes using topical delivery vehicles, including hydrogels, which incorporate active agents such as organic acids, for the treatment of dermal conditions.

Treatment time is typically six months for fingernails, and nine to twelve months for toenails, but this form of treatment has low cure rates, with reported rates being, for example, about 8.5% for a treatment containing cyclopirox and about 46% for a treatment containing amorolfine. Some of the treatments require complete removal of the nail before application of the varnish or lacquer.

US 2004/096410 discloses an alternative topical composition for the treatment of fungal nail infection, which composition comprises a matrix material that incorporates one or more active agents with moisture and one or more humectants. The matrix material is applied to a nail during treatment by cutting the matrix to the approximate size of the nail, placing the matrix on the nail, and securing it to the nail.

A number of the treatments require the nail bed to be debrided prior to application of the varnish or lacquer. For example, the treatment ClearZal® BAC, which contains 0.1% w/w benzalkonuim chloride, provides a nail file to debride the nail prior to application of the liquid. Whilst this debridement may increase efficacy, the re-use of the file will promote the spread of the infection to other nails (or other people) via transfer or spores from the infected nail to a non-infected or recently cured nail.

More recent developments have focused on what are termed "Medical devices", that is devices which, when applied to the human body, have a physical or mechanical effect rather than a biochemical or pharmaceutical effect. The medical devices are claimed to work via the formation of a film over the surface of the nail and penetration of an inhospitable environment to the source of the infection underneath the nail.

There is little clinical evidence that any of these devices work and since most of the fungal nail medical devices available have no means to debride the top-most layer of the nail plate (dorsal layer), penetration of the inhospitable environment and thus an effective treatment, will be extremely limited. Whilst debridement means (nail files) may be available separately, there is no incentive to avoid or minimise the cross-use of the nail files to avoid cross-contamination.

We have appreciated that there is a need for a fungal nail treatment that incorporates a means for: protecting the nail from further exposure to dermatophytes; providing an environment inhospitable to dermatophytes, enhancing the penetration of the inhospitable environment to where it is required; and whereby the means reduces or eliminates the risk of the spread of the infection to other nails.

Having appreciated the above, we have now devised a way of meeting the above needs.

According to an aspect of the invention, there is provided a fungal treatment composition, especially a fungal nail treatment composition as defined in the claims, comprising a proton source. Preferably the composition is a liquid. It will be understood the term "fungal nail treatment composition" implies that, in addition to a proton source, the composition includes other suitable components where necessary for the proper functioning of the composition.

The composition of the present invention consists essentially of a proton source, a humectant, a film forming agent, a penetration enhancing agent, at least one solvent, and optionally at least one additional component selected from the group consisting of one or more nail conditioners, preservatives, UV inhibitors, pigments, dyes and perfumes, as defined in the claims.

According to another aspect of the invention, there is provided a process for the preparation of a fungal nail treatment composition according to the invention, which process comprises providing a proton source, and mixing it with the other suitable components to form a fungal nail treatment composition.

Suitable components include a humectant, a film forming agent, a penetration enhancing agent, at least one solvent, and optional additional components selected from the group consisting of one or more nail conditioners, preservatives, UV inhibitors, pigments, dyes and perfumes.

A composition according to the present invention is preferably a topical composition. According to yet another aspect of the invention, there is provided a process for the preparation of a fungal nail treatment composition according to the invention which process comprises mixing a proton source, a humectant, a film forming agent, a penetration enhancing agent and a solvent so as to form the fungal nail treatment composition.

According to yet another aspect of the invention, there is provided a composition as defined in the claims, for use in the treatment of fungal infection, particularly fungal nail.

According to yet another aspect of the invention, there is provided a treatment composition of the invention in conjunction with a fungal nail treatment device.

According to another aspect of the invention, there is provided a kit, particularly a fungal nail treatment kit, comprising a fungal nail treatment device and a fungal nail treatment composition of the invention.

The present invention provides numerous advantages over prior art methods and compositions for treating fungal nail infection. The composition of the present invention provides a topical fungal treatment composition that does not have the side effects or problems known in the art associated with systemic fungal treatment compositions. The invention is also an improvement upon prior art topical compositions used in the treatment of fungal nail. It can be shown that the composition of the present invention has an improved pharmacological effect over other topical fungal nail treatment compositions such as Loceryl®. The proton source, which serves as an active ingredient in the composition of the invention, provides an improvement over typical topical anti-fungal active agents known from the prior art, which are usually more complex organic compounds. Anti-fungal complex organic compounds used in the prior art are known to have certain negative side affects. Use of the composition of the invention provides for the avoidance of these side effects. Additionally, the composition of the present invention is easier to use than the composition of US 2004/096410 since it does not include a matrix material that needs to be secured to the nail.

The proton source in the fungal nail treatment composition of the present invention is citric acid. The proton source suitably provides that the pH. of the nail treatment composition is in the range of from 0 to 7, preferably from 2 to 6, more preferably from 3 to 5, and most preferably from 2 to 4. The proton source is present in the fungal nail treatment composition in an amount of from 5 to 15% by weight of the treatment fluid composition.

In preferred embodiments of the present invention, the nail treatment composition comprises glycerine as humectant in the form of hygroscopic material. The humectant is present in an amount of from 15 to 40% by weight of the fungal nail treatment fluid composition. It is believed that the hygroscopic humectant absorbs water from the atmosphere and hydrates the nail plate to create a moisture diffusion gradient between the nail plate and the nail bed, to facilitate the diffusion of water-soluble components of the treatment composition to the nail bed. It is also believed that the humectant enhances the effect of a keratolytic agent if present in the composition as it breaks down and softens the nail plate. The protons from the proton source are thought to diffuse freely through the nail plate as it hydrates and softens, travelling along the moisture diffusion gradient created by the water and glycerine, such that the protons accumulate at the nail bed where the infection is principally sited.

The fungal nail treatment fluid composition comprises a film forming agent which is Xanthan gum. The film forming agent is present in an amount of from 0.1 to 1% by weight of the fungal nail treatment fluid composition.

The fungal nail treatment composition comprises a penetration enhancing agent which is urea. The penetration enhancing agent is an agent that helps soften the inhospitable environment that is the nail and helps other components of the fungal nail treatment composition penetrate through the inhospitable environment towards the nail bed. Also disclosed are thioglycolic acid, sodium thioglycolate or potassium thioglycolate. The penetration enhancer is present in an amount of from 1 to 5% by weight of the fungal nail treatment fluid composition.

The fungal nail treatment composition comprises a solvent which is water (e.g. deionised water).

In various embodiments of the present invention, the fungal nail treatment compositions may comprise other suitable components, such as those known in the art of fungal nail treatment compositions. For example, in various embodiments, the fungal nail treatment composition may comprise one or more preservatives, UV inhibitors, pigments, dyes and perfumes.

Preferred additional preservatives may comprise sodium benzoate, sodium sorbate, sorbic acid or benzoic acid. If a preservative is present in the fungal nail treatment composition, it is present in an amount if from 0.1 to 0.5% by weight of the fungal nail treatment composition.

The fungal nail treatment fluid composition comprises a nail conditioner which is panthenol and which is present in an amount from 0.1 to 1% by weight of the treatment fluid composition. If a UV inhibitor is present in the fungal nail treatment composition, it is present in an amount of 0.01 to 0.1% by weight of the fungal treatment fluid composition.

Pigments, dyes and perfumes may be used in the fungal nail treatment composition of the present composition to improve the colour and scent of the composition.

The fungal nail treatment of the present invention comprises at least one additional solvent selected from isopropyl alcohol, ethanol or acetone. The additional solvent is present in an amount of 15 to 40%.

The fungal nail treatment composition of the present invention, comprises citric acid, water, xanthan gum, urea and glycerine.

In a preferred embodiment of the present invention, the amounts of the different components are in accordance with Table 1 below.

**Table 1**

| Function | % w/w |
|---|---|
| Penetration enhancer | 1-5 |
| Humectant | 15-40 |
| Film forming agent | 0.1-1.0 |
| pH adjuster/proton source | 5-15 |
| Preservative | 0.01-0.5 |
| Nail conditioner | 0.1-1 |
| UV inhibitor | 0.01-0.1 |
| Solvent | To 100 |

A particularly preferred composition is given in Table 2 below.

**Table 2**

| Component | Function | %w/w |
|---|---|---|
| Urea | Penetration enhancer | 4.50 |
| Glycerine | Humectant | 20.00 |
| Isopropyl alcohol | Solvent | 20.00 |
| Xanthan gum | Film forming agent | 0.3 |
| Citric acid | pH adjuster/proton source | 12.00 |
| Sodium benzoate | Preservative | 0.2 |
| Panthenol | Nail conditioner | 1.00 |
| Tinogard HS* | UV inhibitor | 0.05 |
| Water | Solvent | 41.95 |

| | | |
|---|---|---|
| *Tinogard® HS is sodium benzotriazolyl butylphenol sulphonate available from BASF, Germany. | | |

Without being limited by theory, when a film forming agent is present in the compositions of the present invention, it is believed the film-forming agent forms a protective film over the whole surface of the infected nail. The film-forming agent also acts a reservoir for the other ingredients of the composition. When a penetration enhancing agent is present in the compositions of the present invention, it is believed that the penetration enhancing agent serves by breaking down and denaturing the keratin molecules that form part of the nail. In this way, it breaks down and softens the inhospitable environment that is the nail and aids other components of the composition in penetrating the inhospitable environment. When a humectant is present in compositions of the present invention, it is believed that the hygroscopic humectant absorbs water from the atmosphere and hydrates the nail plate to create a moisture diffusion gradient between the nail plate and the nail bed, to facilitate the diffusion of water-soluble components of the treatment composition to the nail bed. It is also believed that the humectant enhances the effect of a keratolytic agent if present in the composition as it breaks down and softens the nail plate. The protons from the proton source are thought to diffuse freely through the nail plate as it hydrates and softens, travelling along the moisture diffusion gradient created by the water and glycerine, such that the protons accumulate at the nail bed where the infection is principally sited. This creates a mildly acidic environment that the dermatophytes find inhospitable. It is believed that in this mildly acidic environment, the dermatophytes are not able to generate key components of the cellular structure that are essential to their survival. Thus they die out and the nail discolouration grows out - that is, the new nail growing from the nail bed will be free of fungal infection if it was present.

The method of the present invention comprises mixing the proton source with the other components of the fungal nail treatment composition as described in the preceding paragraphs, and in any of the amounts as described in the preceding paragraphs. The components can be mixed, for example, using conventional methods in the art. The components can be mixed in any order to form the fungal nail treatment composition.

Preferably, the method of the present invention comprises mixing the proton source with a humectant and any of the components described in preceding paragraphs to form a fungal nail treatment composition. More preferably, the method of the present invention comprises mixing a proton source with a humectant, a penetration enhancing agent, a film forming agent and a solvent to form the fungal nail treatment composition of the present invention.

A composition for use in the treatment of fungal infection is preferably a composition according to the invention.

Preferably, the composition is a fungal nail treatment composition, preferably for use topically. The fungal nail treatment composition can be applied to any part of the fingernail or toenail, depending on the site of fungal infection. Preferably, the fungal nail treatment composition is applied to the uppermost layer of the nail plate. The fungal nail treatment composition may be used to treat any form of fungal infection. Preferably, the fungal infection for treatment is an infection of the finger nail or toenail, and more preferably of the nail bed of the fingernail or toenail. The fungal infection may be an infection by any type of fungus. Preferably, the fungal infection is an infection by dermatophytes, and most preferably the infection is an infection by a fungus of the species *Trichophyton rubrum* or *Trichophyton mentagrophytes.*

The fungal nail treatment composition may be applied to the fingernail or toenail in any way. Preferably, before the fungal nail treatment composition is applied to the fingernail or toenail, the upper layer of the nail plate is removed by debridement, before the fungal nail treatment composition is then applied to the new uppermost layer of the nail plate. Without being limited by theory, the process of debridement is designed to reduce the width of the inhospitable environment. This is designed to enhance the penetration of the components of the fungal nail treatment composition through the inhospitable environment so that it comes into contact with the site of infection on the nail plate more easily.

If carried out, the debridement can be carried out by any suitable device. Preferably, the debridement is carried out by a fungal nail treatment device. The fungal nail treatment device for use with the fungal nail treatment composition can be any fungal nail treatment device known in the art, although preferably the device is as described in our co-pending UK patent application (no. 1003340.5).

Whether debridement of the fingernail/toenail is carried out or not during the treatment, the fungal nail treatment composition needs to be applied to the fingernail/toenail. The fungal nail treatment composition for use can be applied by any means known in the art. Preferably, the fungal nail treatment composition is applied using a fungal nail treatment fluid applicator. This can be any suitable or appropriate applicator, but is preferably a brush. Preferably the composition is applied so as to form a continuous film upon the nail.

The fungal nail treatment composition of the present invention may be formulated to have a mode of action designed to kill nail fungus within a period of 1 week to three months, more preferably 1 week to 2 months, even more preferably 2 weeks to 6 weeks, and most preferably in about 28 days. The fungal nail treatment composition may be formulated to carry out the effects mentioned above by preparation of any of the compositions described in previous paragraphs, by any of the methods described in previous paragraphs. Generally, the formulation of the composition does not differ significantly whether the composition is designed to kill nail fungus within 1 week or within 3 months. It is the dosage regime that differs depending on the intended time frame in which to treat the fungal nail infection. Any effective dosage regime for getting rid of the fungal nail infection can be used with the compositions of the present invention. Preferably, a nail treatment (treatment being a debridement of the uppermost layer of the nail plate, followed by application of the composition) is carried out of from three times daily to once every two weeks, more preferably from twice daily to once a week, and most preferably once a day. When a nail treatment with the composition of the invention is applied on a daily basis, the kill rate of the fungus is generally about 99% over a three week period, with complete removal of the fungal infection occurring within a 28 day period. When a weekly treatment is applied, the kill rate of the nail fungus over a three week period is about 75 to 80%, with complete removal of the fungal infection occurring not long after.

The fungal nail treatment is believed to stop dermatophytic metabolism of nail tissue and may prevent further build-up of discoloured nail material over time. The fungal nail treatment composition may also protect the nail from re-infection. Continued use of the fungal nail treatment composition over the treatment time may allow the nail to grow-out and return to a healthy appearance, and removal of the dermatophytes from the site of infection (disappearance of the infection).

The following examples are tests carried out to demonstrate the pharmacological effects of certain fungal nail treatment fluid compositions according to the invention.

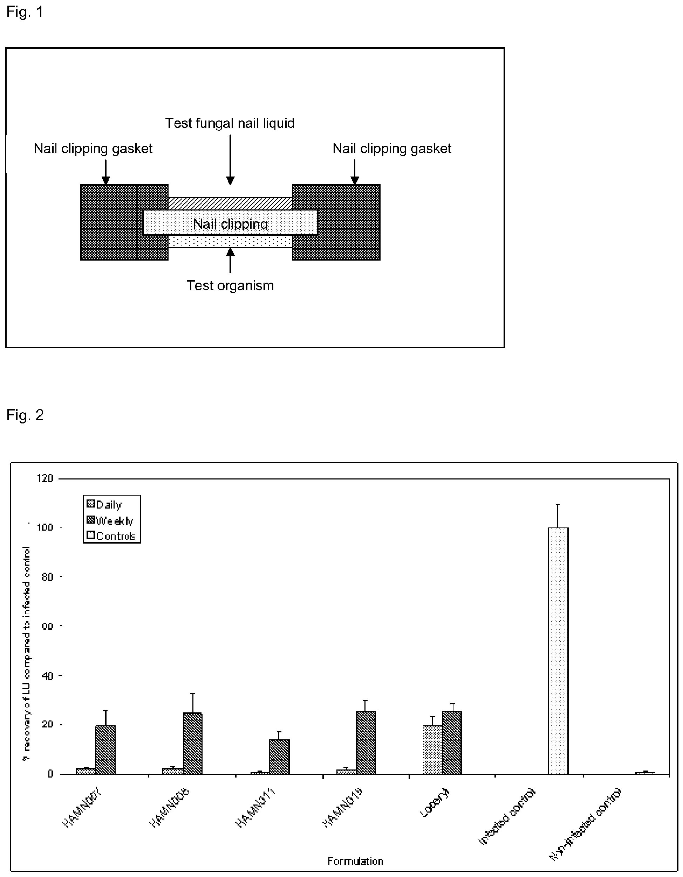

The effectiveness of each fungal nail treatment composition was tested using MedPharm Limited's (Guildford, UK) proprietary *in vitro* test model using human nails infected with *Trichophyton rubrum.* A schematic representation of the test cells used is shown in Figure 1 and the outline of the test is:
1. Full thickness nail clippings, of about 3mm × 3mm in size, were washed and dried and then mounted in the test cell.
2. They were then infected on one side using *Trichophyton rubrum* and incubated for 14 days.
3. The test liquids [see Table 3 for formulations] were then added to the infected nail clippings on the side opposite to that inoculated with *Trichophyton rubrum.*
4. The dosing regime is shown in the table below in Table 4.
5. At the end of the study, the cells were dismantled and the nail clippings analysed for the presence of ATP (adenosine-5'-triphosphate).

**Table 3: Fungal nail treatment liquid formulations tested (reference example)**

| Ingredient | HAMN007 | HAMN 008 | HAM 011 | HAMN 015 |
|---|---|---|---|---|
| | % w/w | % w/w | % w/w | % w/w |
| Water | to 100 | | | |
| Xanthan gum | 0.3 | 0.3 | 0.3 | 0.3 |
| Glycerine | 20 | 20 | 20 | 20 |
| Isopropyl alcohol | 20 | 20 | 20 | 20 |
| Panthenol | 1.0 | 1.0 | 1.0 | 1.0 |
| Citric acid monohydrate | 12 | 12 | 12 | 12 |
| Sodium Benzoate | 0.2 | 0.2 | 0.2 | 0.2 |
| Urea | - | - | - | - |
| Thioglycolic acid | 5.0 | 1.0 | - | - |
| Potassium thioglycolate | - | - | - | - |
| Tinogard HS | 0.05 | 0.05 | 0.05 | 0.05 |

**Table 4: Dosing regime used with fungal nail test cell (reference example)**

| Test item | Formulation | Dosing |
|---|---|---|
| 1 | HAMN 007 | |
| 2 | HAMN 008 | |
| 3 | HAMN 011 | Daily dosing |
| 4 | HAMN 015 | |
| 5 | Loceryl® | |
| 1 | HAMN 007 | |
| 2 | HAMN 008 | |
| 3 | HAMN 011 | |
| 4 | HAMN 015 | Weekly dosing |
| 5 | Loceryl® | |
| Controls | Infected control | |
| | Non-infected control | No dosing |

Tests were conducted against infected and non-infected controls, and against a commercial treatment Loceryl® (Galderma (UK) Ltd) that is in the form of a nonaqueous lacquer and contains 5% w/v amorolfine (an antifungal agent) as the principal active.

For "Daily dosing", each nail clipping was filed using an individual file once a week (days 1, 8 and 15) and the test formulation applied using a brush immediately after filing, ensuring that the whole surface of the nail clipping was covered. Only one coat was applied per application. Each nail clipping was then allowed to dry for five minutes to allow the film to form and the nail clipping was then incubated. The dosing was repeated once a day for 21 days.

For "Weekly dosing", each nail clipping was filed using an individual file once a week (days 1, 8 and 15) and the test formulation applied using a nail brush immediately after filing, ensuring that the whole surface of the nail clipping was covered. Only one coat was applied per application. Each nail clipping was then allowed to dry for five minutes to allow the film to form and was then incubated. The dosing was repeated once a week for 21 days (days 1, 8 and 15).

ATP is a nucleotide produced by living organisms that is used in cells as a coenzyme and transports chemical energy within the cells for metabolism. Its presence is therefore an indication of living organisms and its level can be used to correlate the number of viable organisms present in a sample. The level of ATP was measured using a luminescence technique on the basis that there is a linear correlation between ATP level and luminescence. With the test samples, the luminescence was compared to that of the infected control in terms of the % recovered.

Figure 2 shows the test results for the reference example fungal nail treatment composition formulations detailed in Table 3, tested according to the dosing regimes detailed in Table 4.

Figure 2 clearly shows that the four fungal nail treatment liquids of the invention are much more effective against fungal nail than Loceryl® treatment when used on a daily basis. When applied on a weekly basis, the fungal nail treatment compositions of the invention have a kill rate of about 80% when applied weekly, and a kill rate of about 99% when applied daily, when applied over a period of three weeks. In comparison, the kill rate for Loceryl applied under the same regime, ranges from about 75% to 80%.

Preferably, the fungal nail treatment fluid of the present invention is used to treat fungal infection of the fingernail/toenail in conjunction with a fungal nail treatment device.

The fungal nail treatment device for use with the fungal nail treatment composition may be any fungal nail treatment device known in the art. In a preferred embodiment, the fungal nail treatment device comprises disposable nail files along with the fungal nail treatment fluid composition. If the device comprises nail files, these nail files are preferably spring-loaded. If the device comprises spring-loaded nail files, these may be comprised in the fungal nail treatment device in any way. Preferably, the spring-loaded nails files are housed in the fungal nail treatment device with a one-way dispensing mechanism. The fungal nail treatment device may also comprise an applicator. The applicator may be housed anywhere within the fungal nail treatment device. Preferably, the applicator is housed in the fungal nail treatment device in a container attached to the dispensing mechanism. The fungal nail treatment device preferably comprises a container for containment of the fungal nail treatment composition. The container may be attached or housed in the fungal nail treatment device in/by any way. Preferably, this container for containment of the fungal nail treatment composition is attached to the dispensing mechanism.

In a preferred embodiment of the fungal nail treatment device comprising the fungal nail treatment composition, the fungal nail treatment device comprises disposable nail files that are provided in a spring-loaded dispensing mechanism that is one-way, making it impossible for the user to place a contaminated file back in the dispensing mechanism. This has the benefit of educating the consumer to dispose of the file after use, and therefore not spreading the condition by subsequent re-use of a file that is carrying dermatophyte hyphae or spores.

If the device comprises disposable nail files, the number of disposable nail files in the device is preferably 1 to 10, more preferably 2 to 7 and most preferably 5. The disposable nail files may be coated with any substance suitable to coat nail files in. The coating may serve to help the nail file perform its function of removing the upper surface of the nail plate. The coating may also serve to be toxic to any fungi living within the nail plate. Or the coating of the nail file may perform both of these functions. In a preferable embodiment, the disposable nail files are coated with carborundum (silicon carbide).

The fungal nail treatment composition that is comprised within the fungal nail treatment device may comprise any of the components/groupings of components described in preceding paragraphs, and the various components may be present in any of the amounts described in preceding paragraphs, in the fungal nail treatment fluid composition for use in conjunction with the fungal nail treatment device.

The fungal nail treatment composition for use in the treatment of fungal infection in conjunction with the fungal nail treatment device may comprise any of the components described in preceding paragraphs, and the various components may be present in any of the amounts described in preceding paragraphs, in the fungal nail treatment fluid composition for use in conjunction with the fungal nail treatment device.

The fungal nail treatment device for use with the fungal treatment composition for use in the methods of the present invention may be any fungal nail treatment device known in the art. Any of the fungal nail treatment devices described in this application may be used in conjunction with any of the fungal nail treatment compositions described in this application in the methods of the present invention.

Preferably, the topical application of the treatment composition is application to the top of the nail plate to form a film. In a preferable embodiment of use, the uppermost layer of the nail plate is debrided prior to application of the fungal nail treatment fluid composition. The debridement is preferably carried out by the abrasive action of a disposable nail file. Without being limited by theory, the removal of the uppermost layer of the nail plate has a number of benefits including a visual improvement in the appearance of the nail, the removal of some of the dermatophytes present in the nail plate, and a reduction in the distance that the fungal nail treatment liquid needs to travel or diffuse through to reach the source of the infection, and to create an inhospitable environment against it.

In a preferred embodiment, once the debridement has occurred, the fungal nail treatment liquid is applied using an applicator, which can be a brush or any suitable applicator. The fungal nail treatment fluid composition is applied to the upper surface of the nail to forma continuous film on it as it dries. This helps prevent the spread of infection from the infected nail, and also helps protect the debrided nail from re-infection.

The fungal nail treatment composition can then act topically against the dermatophytes causing the infection to subside by any means. Preferably, this is achieved by the fungal nail treatment composition penetrating through the nail plate to the source of the infection, creating an inhospitable environment against it, and leaving the dermatophytes to die.

## Claims

1. A fungal nail treatment composition consisting essentially of:
5-15wt% citric acid as an active agent proton source;
15-40wt% glycerine as a humectant in the form of hygroscopic material;
0.1-1.0wt% xanthan gum as a film forming agent;
1-5wt% urea as a penetration enhancing agent;
water up to 100wt% of the total composition and at least one of isopropyl alcohol, ethanol or acetone as solvents in an amount 15 to 40wt%;
0.1-1.0wt% panthenol as a nail conditioner;
and, optionally, 0.01-0.1wt% UV inhibitor and/or 0.1-0.5wt% preservative and/or pigments and/or dyes and/or perfumes.

2. A fungal nail treatment composition according to claim 1 wherein the composition consists of:
5-15wt% citric acid as an active agent proton source;
15-40wt% glycerine as a humectant in the form of hygroscopic material;
0.1-1.0wt% xanthan gum as a film forming agent;
1-5wt% urea as a penetration enhancing agent;
water up to 100wt% of the total composition and at least one of isopropyl alcohol, ethanol or acetone as solvents in an amount 15 to 40wt%;
0.1-1.0wt% panthenol as a nail conditioner;
and, optionally, 0.01-0.1wt% UV inhibitor and/or 0.1-0.5wt% preservative and/or pigments and/or dyes and/or perfumes.

3. A fungal nail treatment composition according to claim 1, wherein the composition consists essentially of:
12wt% citric acid as an active agent proton source;
20wt% glycerine as a humectant in the form of hygroscopic material;
0.3wt% xanthan gum as a film forming agent;
4.5wt% urea as a penetration enhancing agent;
41.95wt% water and 20wt% isopropyl alcohol as solvents;
1.0wt% panthenol as a nail conditioner;
and 0.05wt% sodium benzotriazolyl butylphenol sulphonate as UV inhibitor and 0.2wt% sodium benzoate as preservative.

4. A fungal nail treatment composition according to claim 1, wherein the composition consists of:
12wt% citric acid as an active agent proton source;
20wt% glycerine as a humectant in the form of hygroscopic material;
0.3wt% xanthan gum as a film forming agent;
4.5wt% urea as a penetration enhancing agent;
41.95wt% water and 20wt% isopropyl alcohol as solvents;
1.0wt% panthenol as a nail conditioner;
and 0.05wt% sodium benzotriazolyl butylphenol sulphonate as UV inhibitor and 0.2wt% sodium benzoate as preservative.

5. A process for the preparation of fungal nail treatment composition according to claim 1, comprising the steps of mixing:
5-15wt% citric acid as an active agent proton source;
15-40wt% glycerine as a humectant in the form of hygroscopic material;
0.1-1.0wt% xanthan gum as a film forming agent;
1-5wt% urea as a penetration enhancing agent;
water up to 100wt% of the total composition and at least one of isopropyl alcohol, ethanol or
acetone as solvents in an amount 15 to 40wt%;
0.1-1.0wt% panthenol as a nail conditioner;
and, optionally, 0.01-0.1wt% UV inhibitor and/or 0.1-0.5wt% preservative and/or pigments and/or dyes and/or perfumes; to provide said composition.

## Patentansprüche

1. Zusammensetzung zur Behandlung von Nagelpilz, im Wesentlichen bestehend aus:
5 bis 15 Gew.-% Zitronensäure als Wirkstoffprotonenquelle;
15 bis 40 Gew.-% Glycerin als Feuchthaltemittel in Form von hygroskopischem Material;
0,1 bis 1,0 Gew.-% Xanthan als Filmbildner;
1 bis 5 Gew.-% Harnstoff als penetrationsverstärkendem Mittel;
Wasser bis zu 100 Gew.-% der Gesamtzusammensetzung und mindestens einem aus Isopropylalkohol, Ethanol oder Aceton als Lösungsmittel in einer Menge von 15 bis 40 Gew.-%;
0,1 bis 1,0 Gew.-% Panthenol als Nagelpflegemittel;
und wahlweise 0,01 bis 0,1 Gew.-% UV-Inhibitor und/oder 0,1 bis 0,5 Gew.-% Konservierungsmittel und/oder Pigmenten und/oder Farbstoffen und/oder Duftstoffen.

2. Zusammensetzung zur Behandlung von Nagelpilz nach Anspruch 1, wobei die Zusammensetzung besteht aus:
5 bis 15 Gew.-% Zitronensäure als Wirkstoffprotonenquelle;
15 bis 40 Gew.-% Glycerin als Feuchthaltemittel in Form von hygroskopischem Material;
0,1 bis 1,0 Gew.-% Xanthan als Filmbildner;
1 bis 5 Gew.-% Harnstoff als penetrationsverstärkendem Mittel;
Wasser bis zu 100 Gew.-% der Gesamtzusammensetzung und mindestens einem aus Isopropylalkohol, Ethanol oder Aceton als Lösungsmittel in einer Menge von 15 bis 40 Gew.-%;
0,1 bis 1,0 Gew.-% Panthenol als Nagelpflegemittel;
und wahlweise 0,01 bis 0,1 Gew.-% UV-Inhibitor und/oder 0,1 bis 0,5 Gew.-% Konservierungsmittel und/oder Pigmenten und/oder Farbstoffen und/oder Duftstoffen.

3. Zusammensetzung zur Behandlung von Nagelpilz nach Anspruch 1, wobei die Zusammensetzung im Wesentlichen besteht aus:
12 Gew.-% Zitronensäure als aktiver Protonenquelle;
20 Gew.-% Glycerin als Feuchthaltemittel in Form von hygroskopischem Material;
0,3 Gew.-% Xanthangummi als Filmbildner;
4,5 Gew.-% Harnstoff als penetrationsverstärkendem Mittel;
41,95 Gew.-% Wasser und 20 Gew.-% Isopropylalkohol als Lösungsmittel;
1,0 Gew.-% Panthenol als Nagelpflegemittel;
und 0,05 Gew.-% Natriumbenzotriazolylbutylphenolsulfonat als UV-Inhibitor und 0,2 Gew.-% Natriumbenzoat als Konservierungsmittel.

4. Zusammensetzung zur Behandlung von Nagelpilz nach Anspruch 1, wobei die Zusammensetzung besteht aus:
12 Gew.-% Zitronensäure als aktiver Protonenquelle;
20 Gew.-% Glycerin als Feuchthaltemittel in Form von hygroskopischem Material;
0,3 Gew.-% Xanthangummi als Filmbildner;
4,5 Gew.-% Harnstoff als penetrationsverstärkendem Mittel;
41,95 Gew.-% Wasser und 20 Gew.-% Isopropylalkohol als Lösungsmittel;
1,0 Gew.-% Panthenol als Nagelpflegemittel;
und 0,05 Gew.-% Natriumbenzotriazolylbutylphenolsulfonat als UV-Inhibitor und 0,2 Gew.-% Natriumbenzoat als Konservierungsmittel.

5. Verfahren zur Herstellung einer Zusammensetzung zur Behandlung von Nagelpilz nach Anspruch 1, umfassend die Schritte des Mischens von:
5 bis 15 Gew.-% Zitronensäure als Wirkstoffprotonenquelle;
15 bis 40 Gew.-% Glycerin als Feuchthaltemittel in Form von hygroskopischem Material;
0,1 bis 1,0 Gew.-% Xanthan als Filmbildner;
1 bis 5 Gew.-% Harnstoff als penetrationsverstärkendem Mittel;
Wasser bis zu 100 Gew.-% der Gesamtzusammensetzung und mindestens einem aus Isopropylalkohol, Ethanol oder Aceton als Lösungsmittel in einer Menge von 15 bis 40 Gew.-%;
0,1 bis 1,0 Gew.-% Panthenol als Nagelpflegemittel;
und wahlweise 0,01 bis 0,1 Gew.-% UV-Inhibitor und/oder 0,1 bis 0,5 Gew.-% Konservierungsmittel und/oder Pigmenten und/oder Farbstoffen und/oder Duftstoffen, um die Zusammensetzung bereitzustellen.

## Revendications

1. Composition de traitement fongique pour les ongles comprenant essentiellement :
5 à 15 % en poids d'acide citrique comme source de protons d'agent actif ;
15 à 40 % en poids de glycérine comme humectant sous forme de matériau hygroscopique ;
0,1 à 1,0 % en poids de gomme de xanthane comme agent filmogène ;
1 à 5 % en poids d'urée comme agent améliorant la pénétration ;
de l'eau jusqu'à 100 % en poids de la composition totale et au moins un parmi l'alcool isopropylique, l'éthanol ou l'acétone comme solvants dans une quantité de 15 à 40 % en poids ;
0,1 à 1,0 % en poids de panthénol comme revitalisant pour les ongles ;
et, éventuellement, 0,01 à 0,1 % en poids d'inhibiteur d'UV et/ou 0,1 à 0,5 % en poids de conservateur et/ou de pigments et/ou de colorants et/ou de parfums.

2. Composition de traitement fongique pour les ongles selon la revendication 1, la composition comprenant :
5 à 15 % en poids d'acide citrique comme source de protons d'agent actif ;
15 à 40 % en poids de glycérine comme humectant sous forme de matériau hygroscopique ;
0,1 à 1,0 % en poids de gomme de xanthane comme agent filmogène ;
1 à 5 % en poids d'urée comme agent améliorant la pénétration ;
de l'eau jusqu'à 100 % en poids de la composition totale et au moins un parmi l'alcool isopropylique, l'éthanol ou l'acétone comme solvants dans une quantité de 15 à 40 % en poids ;
0,1 à 1,0 % en poids de panthénol comme revitalisant pour les ongles ;
et, éventuellement, 0,01 à 0,1 % en poids d'inhibiteur d'UV et/ou 0,1 à 0,5 % en poids de conservateur et/ou de pigments et/ou de colorants et/ou de parfums.

3. Composition de traitement fongique pour les ongles selon la revendication 1, la composition comprenant essentiellement :
12 % en poids d'acide citrique comme source de protons d'agent actif ;
20 % en poids de glycérine comme humectant sous forme de matériau hygroscopique ;
0,3 % en poids de gomme de xanthane comme agent filmogène ;
4,5 % en poids d'urée comme agent améliorant la pénétration ;
41,95 % en poids d'eau et 20 % en poids d'alcool isopropylique comme solvants ;
1,0 % en poids de panthénol comme revitalisant pour les ongles ;
et 0,05 % en poids de benzotriazolyl butylphénol sulfonate de sodium comme inhibiteur d'UV et 0,2 % en poids de benzoate de sodium comme conservateur.

4. Composition de traitement fongique pour les ongles selon la revendication 1, la composition comprenant :
12 % en poids d'acide citrique comme source de protons d'agent actif ;
20 % en poids de glycérine comme humectant sous forme de matériau hygroscopique ;
0,3 % en poids de gomme de xanthane comme agent filmogène ;
4,5 % en poids d'urée comme agent améliorant la pénétration ;
41,95 % en poids d'eau et 20 % en poids d'alcool isopropylique comme solvants ;
1,0 % en poids de panthénol comme revitalisant pour les ongles ;
et 0,05 % en poids de benzotriazolyl butylphénol sulfonate de sodium comme inhibiteur d'UV et 0,2 % en poids de benzoate de sodium comme conservateur.

5. Procédé de préparation d'une composition de traitement fongique pour les ongles selon la revendication 1, comprenant les étapes consistant à mélanger :
5 à 15 % en poids d'acide citrique comme source de protons d'agent actif ;
15 à 40 % en poids de glycérine comme humectant sous forme de matériau hygroscopique ;
0,1 à 1,0 % en poids de gomme de xanthane comme agent filmogène ;
1 à 5 % en poids d'urée comme agent améliorant la pénétration ;
de l'eau jusqu'à 100 % en poids de la composition totale et au moins un parmi l'alcool isopropylique, l'éthanol ou l'acétone comme solvants dans une quantité de 15 à 40 % en poids ;
0,1 à 1,0 % en poids de panthénol comme revitalisant pour les ongles ;
et, éventuellement, 0,01 à 0,1 % en poids d'inhibiteur d'UV et/ou 0,1 à 0,5 % en poids de conservateur et/ou de pigments et/ou de colorants et/ou de parfums ; pour former ladite composition.
